# FASCICULE DE BREVET EUROPEEN

(11) **EP 2 509 588 B1**
(45) Date de publication et mention de la délivrance du brevet: **01.05.2019**
(21) Numéro de dépôt: 10801645.2
(22) Date de dépôt: 09.12.2010
(51) Int. Cl.: A61K 9/50, A23L 33/105, A23L 33/12, A23L 33/15, A23L 33/155, A23L 33/16, A23K 40/30, A23K 20/174, A23K 20/24

(54) **PARTICULES DE PRINCIPES ACTIFS LIPOSOLUBLES STABLES**
STABILE FETTLÖSLICHE WIRKSTOFFPARTIKEL
STABLE FAT-SOLUBLE ACTIVE PRINCIPLE PARTICLES

(30) Priorité: 09.12.2009 FR 0958779
(43) Date de publication de la demande: 17.10.2012
(73) Titulaire: Adisseo France S.A.S., 92160 Antony (FR)
(72) Inventeur: DOLLAT, Jean-Marie, F-03100 Montluçon (FR); DAFFIS, Bernard, F-03310 Neris les Bains (FR)
(74) Mandataire: Verriest, Philippe
(86) Numéro de dépôt international: PCT/FR2010/052656
(87) Numéro de publication internationale: WO 2011/070300

(56) Documents cités:
- EP-A1- 0 633 058
- EP-A2- 0 494 417
- WO-A1-2004/057980
- US-A- 5 153 177
- US-B1- 6 328 995
- CORTESI R ET AL: "Sugar cross-linked gelatin for controlled release: microspheres and disks", BIOMATERIALS, ELSEVIER SCIENCE PUBLISHERS BV., BARKING, GB, vol. 19, no. 18, 1 September 1998 (1998-09-01), pages 1641-1649, XP004161435, ISSN: 0142-9612, DOI: 10.1016/S0142-9612(98)00034-9

## Description

La présente invention concerne des principes actifs alimentaires et/ou médicamenteux liposolubles sous la forme de particules qui présentent non seulement une bonne stabilité au stockage, mais aussi en mélange dans un pré-mélange d'additifs qui contient des agents agressifs, destiné tout particulièrement à la nutrition animale.

La présente invention a plus précisément trait à la préparation de composés à base de vitamines et/ou de caroténoïdes, et/ou leurs dérivés de ceux-ci.

Les vitamines et les caroténoïdes sous forme de particules (ou autrement dit de poudres) sont très largement employés dans de nombreux domaines techniques tels que l'industrie pharmaceutique, l'industrie agroalimentaire, et notamment dans le domaine de la nutrition animale. A titre d'exemple, les vitamines A et E sont couramment utilisées pour la préparation d'aliments favorisant la croissance d'animaux. Ces aliments sont cependant préparés au moyen de procédés mettant en oeuvre des conditions extrêmes, telles que des températures, une humidité, des pressions et des tensions mécaniques élevées, qui peuvent être dommageables aux principes actifs, et en particulier aux vitamines, présents dans ces aliments.

Par ailleurs, aussi bien en pharmacie qu'en nutrition animale, il est important que ledit principe actif ne soit pas dégradé dès qu'il rencontre les premières conditions sévères, notamment acides, en entrant dans le système digestif.

C'est pourquoi, afin de préserver au mieux ces principes actifs sensibles à ces conditions extrêmes précitées, il est connu depuis longtemps de les protéger en les mélangeant avec des protéines telles que de la gélatine et en provoquant la réticulation de ces protéines autour de ces principes actifs.

Mais la vitamine A sous forme d'acétate ou la vitamine E sont des produits huileux qui ne se mélangent avec les protéines et leur agent de réticulation que sous la forme d'une émulsion huile dans l'eau qui n'est jamais facile à manipuler.

A cet égard, divers procédés de réticulation des protéines en présence de vitamine A ont été décrits.

Le document EP A 0 261 616 décrit un procédé de réticulation de la gélatine en présence d'acétaldéhyde pour protéger la vitamine A. Plus précisément, on réalise un mélange qui comprend une protéine, un alcool miscible à l'eau, de l'acétaldéhyde, de l'eau et de la vitamine A sous la forme de gouttes de taille inférieure à 10 µm. Ce mélange est lyophilisé de façon à obtenir des particules solides de diamètre compris entre 100 et 800 microns. Les particules solides ainsi obtenues sont ensuite soumises à des vapeurs d'acétaldéhyde pendant une période d'environ 3 heures à une température comprise entre 60 et 90°C. Néanmoins, ce procédé ne peut être réalisé en continu, car il nécessite deux étapes, chacune d'entre elles exigeant un type d'appareillage différent, à savoir un lyophiliseur et un appareil de pulvérisation. La lyophilisation est une étape coûteuse, et dont la productivité est extrêmement limitée.

Aussi, selon le procédé de préparation de sphérules de vitamine A décrit dans le document EP A 0 285 682, il est connu de préparer une émulsion contenant une vitamine, de l'eau, de la gélatine et un sucre que l'on transforme en gouttelettes par pulvérisation. Ces gouttelettes sont ensuite mises en contact individuellement avec une poudre de cellulose présentant des caractéristiques bien définies jusqu'au durcissement des gouttelettes. Les gouttelettes ainsi durcies sont alors séparées de la poudre de cellulose par tamisage, le tamis devant retenir les gouttelettes durcies et laisser passer la poudre ; ce qui nécessite un choix strict de la dimension granulométrique de la poudre de cellulose et surtout de sa nature pour ne pas s'agglomérer durant la mise en oeuvre du procédé. Les gouttelettes récupérées sont ensuite séchées, puis soumises à une opération de chauffage afin d'assurer la réticulation de la gélatine par réaction des groupes aminés de la gélatine avec les fonctions réductrices du sucre. Ce procédé est particulièrement difficile à mettre en oeuvre de par le choix strict des matières utilisées et une surveillance étroite des conditions de mise en oeuvre.

Il ressort des procédés décrits ci-dessus que la réticulation de la protéine exige un chauffage à des températures assez élevées et de surcroît pendant une période relativement longue, ce qui n'est guère compatible avec la stabilité des vitamines dans ces conditions.

Le document EP A 1 088 486 tente de remédier à ces inconvénients en proposant un procédé de préparation de poudres sèches stables contenant des vitamines liposolubles et/ou des caroténoïdes mettant en oeuvre une étape de réticulation dans des conditions moins drastiques, à savoir durant un temps de réaction court, préférentiellement inférieur à une demi-heure. En effet, ce procédé de préparation comprend les étapes suivantes :
- une étape de réalisation d'une dispersion aqueuse comprenant une protéine, un sucre, un sel de phosphate de métal alcalin et la vitamine (ou caroténoïde), suivie
- d'une étape de transformation de cette dispersion en une poudre sèche, notamment par un séchage, puis
- une étape de réticulation consistant en un traitement thermique de la protéine à une température comprise entre 55°C et 180°C, mais préférentiellement entre 85°C et 125°C, pendant une durée comprise entre 5 minutes et 3 heures, de préférence entre 6 et 25 minutes.

Il n'en demeure pas moins que les principes actifs sensibles, tels que les vitamines, peuvent aussi subir des dommages lors de leur mise en mélange avec des agents agressifs tels que des sels métalliques (par exemples les sulfates, les iodates, les carbonates) ou encore des oxydes lors de la préparation de pré-mélange, dits de ce fait « agressifs », et qui sont destinés à la nutrition animale. Il est ainsi aussi indispensable de protéger ces principes actifs sensibles contre les dommages causés par les sulfates tels que les sulfates de cobalt, de cuivre, de fer, les carbonates tels que les carbonates de fer, les iodates tels que les iodates de calcium, ou encore contre certains oxydes tels que l'oxyde vert de manganèse ou les oxydes de zinc ou encore des composés à base de sélénium. En effet, ces agents agressifs peuvent réagir selon des réactions d'oxydoréduction avec les principes actifs tels que les vitamines et donc les dégrader.

La présente invention propose de remédier à ces difficultés de préparation de principes actifs liposolubles, et en particulier de vitamines, qui ont été détaillées dans l'état de la technique ci-dessus. En effet, la présente invention propose un nouveau procédé de préparation de principes actifs liposolubles qui, outre leurs excellentes propriétés de stabilité dans des conditions extrêmes au cours de procédés industriels et lors de leur stockage, présentent une bonne stabilité lors de leur mise en mélange dans des pré-mélange dits « agressif ». En outre, à la différence de certains des procédés de préparation précités, tels que celui décrit dans le document EP A 1 088 486, le procédé de préparation selon la présente invention présente l'avantage de ne pas comporter d'étape de traitement thermique pour réaliser l'étape de réticulation de la protéine mise en oeuvre au cours de ce procédé. En effet, la réticulation peut être effectuée dans des conditions dites « douces », à savoir à des températures peu élevées, de l'ordre de 60°C.

De plus, les principes actifs ainsi obtenus selon l'invention exhibent d'excellentes propriétés d'insolubilité dans une eau chaude à plus de 60°C. Or, comme cela est connu de l'homme de métier, cette propriété d'insolubilité dans une eau chaude est significative que les formulations ainsi obtenues seront tout à fait stables dans les procédés de préparation d'aliments avec des presses à granuler à des températures de 80 à 90° en milieu humide. D'autre part, cette particularité est mise à profit pour l'utilisation des ces formules dans des aliments pour ruminants. En effet, pendant le séjour de plusieurs heures dans le rumen à 40°C, le principe actif reste insoluble et sera libéré dans la partie intestinale.

Le procédé de préparation de principes actifs liposolubles, en particulier pharmaceutiques et/ou alimentaires, sous la forme de particules, comprend les étapes suivantes :
a) on prépare une émulsion huile dans l'eau comprenant en pourcentages en poids par rapport au poids total de ladite émulsion :
   - 8 à 20 % d'au moins une protéine, préférentiellement 10 à 15%,
   - 5 à 15 % d'au moins un sucre, préférentiellement 8 à 12%,
   - 10 à 22% d'au moins un principe actif liposoluble sous forme huileuse et/ou dissous dans une huile alimentaire, préférentiellement 15 à 20%,
   - qsp % d'eau,
b) on procède au formage de particules sous la forme substantiellement sphériques par la dispersion de l'émulsion huile dans l'eau obtenue à l'issue de l'étape a) dans un fluide,
c) on ajoute au moins un agent de réticulation de la protéine à la dispersion obtenue à l'issue de l'étape b), ledit agent de réticulation étant choisi parmi l'acétaldéhyde, le glutaraldéhyde, le glyoxal, de préférence le glutaraldéhyde,
d) on récupère les principes actifs sous la forme substantiellement sphériques,
   - l'étape de réticulation de la protéine est réalisée sans étape de traitement thermique,
   - caractérisé en ce que ladite émulsion de l'étape a) comprend en outre 0,5 à 3%, préférentiellement 2 à 3%, d'un sel inorganique qui est le NaH2P04.

La protéine peut être choisie parmi les protéines végétales ou animales. Par exemple, on peut utiliser de la gélatine, en particulier de la gélatine de peau ou d'os de porc, ainsi que de la gélatine bovine, de la gélatine de poisson. Les gélatines de type A ou B conviennent tout à fait, et de préférence avec une valeur de Bloom comprise entre 50 et 300. La protéine peut être aussi choisie parmi la caséine ou la caséinate, ou encore il peut s'agir de protéines du soja ou du maïs.

Le sucre peut être choisi parmi les polyols, les monosaccharides, les disaccharides, les sirops de glucose et de fructose et les maltodextrines. En particulier, on préfère les glycérols, les sorbitols, les maltitols et xylitol, le fructose, le glucose, le lactose, le maltose, le xylose, le sucrose, l'arabinose, le ribose et le saccharose. Encore plus préférentiellement, on utilise le fructose, le glucose, des sirops de glucose ou encore le saccharose, pris seuls ou en mélanges.

Le sel inorganique est le sodium dihydrogène phosphate (NaH₂PO₄).

Dans le cadre du procédé selon l'invention, on peut utiliser tout principe actif pouvant être dissous ou dispersé dans une huile alimentaire. Par huile alimentaire, on entend aussi bien les huiles alimentaires végétales ou animales. L'huile d'arachide, de tournesol, de colza, de maïs, de soja, de palme ou leurs dérivés esters méthyliques ou encore de foie de morue sont particulièrement préférées.

Les principes actifs liposolubles peuvent être notamment choisis parmi :
- les vitamines telles que la vitamine A, E, D et notamment la vitamine D3, K et notamment la vitamine K3, ainsi que
- les dérivés de ces vitamines tels leurs esters, et notamment les esters de vitamines A comme l'acétate de vitamine A, le propionate de vitamine A ou le palmitate de vitamine A, ou encore les esters de vitamines E comme l'acétate de tocophéryl,
- les caroténoïdes tels que le bêta-carotène, le lycopène, la bixine, la zéaxanthine, la citranaxanthine, l'asthaxanthine, la canthaxanthine, la lutéine, la capsanthine, la cryptoxanthine, l'acide bêta-apo-8'caroténoïque et ses esters, le bêta-apo-8' carotenal, le bêta-apo-12'-caroténal,
- les acides gras polyinsaturés tels que les omégas 3 et omégas 6,

De manière avantageuse, on utilise des vitamines mises en solution dans une huile alimentaire ou bien des provitamines. Les vitamines peuvent être des vitamines pures d'origine naturelle ou synthétique. On préfère tout particulièrement les vitamines sous forme huileuse, en particulier la vitamine E et la vitamine A.

Parmi les caroténoïdes, on préfère le bêta-carotène, le lycopène, la lutéine, la zéaxanthine, la canthaxanthine et l'asthaxanthine.

Lorsque les principes actifs liposolubles sont sensibles à l'oxydation, ce qui est souvent le cas dans le domaine technique de la nutrition animale, il est particulièrement avantageux d'ajouter au moins un antioxydant dans l'émulsion huile dans l'eau de l'étape a). L'agent antioxydant peut être choisi parmi le tertiobutyl 3 hydroxy-4 anisole (BHA), le ditertiobutyl-,5,hydroxy-4-toluène (BHT), l'éthoxy-6dihydroxy-1,2triméthyl-2,2,4quinoléine (éthoxyquinine), le tertiobutyl-2 dihydroxy-1,4 benzène ou encore le tocophérol, l'acide citrique ou l'acide phytique et leurs sels de métaux alcalins, ou encore l'acide éthylène diamine tetra acétique (EDTA).

En outre, l'émulsion huile dans l'eau de l'étape a) du procédé selon l'invention peut comprendre des humidifiants tels que des polyéthylènes glycol, le sorbitol ou le glycérol ou un émulsifiant tel que la lécithine.

Aussi, des épaississants tels que la gomme arabique, la gomme de guar, des alginates ou certains amidons modifiés peuvent être ajoutés dans la l'émulsion huile dans l'eau de l'étape a) afin d'ajuster la viscosité de celle-ci.

La préparation de l'émulsion huile dans l'eau de l'étape a) du procédé selon l'invention peut être réalisée de la manière suivante :
i. Dans un premier mélangeur, on dissout la ou les protéines dans de l'eau chaude à une température comprise entre 45°C et 70°C, de préférence entre 45°C et 55°C, plus préférentiellement à 50°C, et ce sous agitation, par exemple à une vitesse de 2 à 3 m/s et pendant au moins 20 minutes.
ii. On ajoute dans ce premier mélangeur le(s) sucre(s), le(s) sel(s) inorganique(s) et le(s) principe(s) actif(s) liposoluble(s) sous forme huileuse, et éventuellement des antioxydants, ainsi qu'au moins un des constituants détaillés ci-dessus tels que choisis parmi les humidifiants, les émulsifiants, les épaississants. La température est toujours maintenue à une température comprise entre 45°C et 70°C, de préférence entre 45°C et 55°C, plus préférentiellement à 50°C. Le mélange est maintenu agité lors de l'ajout de ces différents constituants, par exemple à une vitesse de 2 à 3 m/s. Afin que le mélange soit tout à fait homogène, l'agitation est maintenue au moins dix minutes.

Une autre alternative à la préparation de l'émulsion huile dans l'eau peut consister à dissoudre dans un premier mélangeur le(s) sucre(s) dans de l'eau chaude à une température comprise entre 45°C et 70°C, de préférence entre 45°C et 55°C, plus préférentiellement à 50°C, puis à y ajouter la ou les protéines, et ce sous agitation et de manière à obtenir un mélange homogène. Ensuite, les autres constituants que sont le(s) sel(s) inorganique(s), le(s) principe(s) actif(s), et les constituants optionnels sont ajoutés successivement au mélange dans ce premier mélangeur.

De plus, de manière tout à fait avantageuse, préalablement à l'étape a), avant d'ajouter les principes actifs dans le premier mélangeur, on dissout dans un second mélangeur les principes actifs dans une huile alimentaire telle que l'huile d'arachide, de tournesol, de colza, de maïs, de soja, de palme ou encore de foie de morue. Et l'on ajoute alors ce mélange obtenu dans ce deuxième mélangeur dans le premier mélangeur.

Ce pré-mélange dans une huile alimentaire contribue à améliorer la stabilité des principes actifs lors de la mise en oeuvre des étapes du procédé selon l'invention, puis lors du stockage du produit obtenu à l'issue dudit procédé. En effet, l'huile alimentaire se présente initialement sous forme liquide. Elle va être amenée à se solidifier au cours du procédé selon l'invention et se trouvera sous forme solide à l'issue dudit procédé, et ce en enrobant lesdits principes actifs ; ce qui aura l'avantage de les protéger au cours du stockage.

L'étape b) du procédé selon l'invention est une étape déterminant la forme des particules de principes actifs qui seront obtenues à l'issue dudit procédé. Une forme particulaire tout à fait avantageuse correspond à une forme la plus sphérique possible et de taille adéquate, à savoir pouvant être comprise entre 50 et 630 µm. Le choix de la taille des particules peut être notamment tributaire du domaine d'application des principes actifs envisagé.

Ainsi, l'étape b) du procédé selon l'invention est réalisée de manière adéquate en fonction de la taille particulaire souhaitée du produit obtenu à l'issue du procédé.

L'émulsion huile dans l'eau obtenue à l'issue de l'étape a) peut être dispersée au cours de l'étape b) du procédé selon l'invention dans de l'air par atomisation, par exemple au sein d'une tour d'atomisation via des buses ou des turbines. Avantageusement, la température d'entrée d'air est d'environ 150°C et l'émulsion est atomisée à une température de l'ordre de 60°C.

Dans un mode de réalisation très avantageux de l'invention, l'émulsion huile dans l'eau obtenue à l'issue de l'étape a) est dispersée au cours de l'étape b) du procédé selon l'invention dans une huile alimentaire de manière à obtenir une « double émulsion », à savoir une émulsion (huile dans l'eau) dans huile. Il peut s'agir d'une huile choisie parmi l'huile d'arachide, de tournesol, de colza, de maïs, de soja, de palme, de leurs dérivés esters méthyliques ou encore de foie de morue.

A l'étape c) du procédé selon l'invention, l'agent de réticulation de la protéine peut être choisi parmi l'acétaldéhyde, le glutaraldéhyde, le glyoxal. On utilise de préférence le glutaraldéhyde. De plus, l'agent de réticulation peut être utilisé à l'état pur ou de préférence en solution aqueuse selon une concentration comprise entre 5 et 25%. L'agent de réticulation réagit avec les groupes aminés de la protéine en formant des liaisons imines. Par exemple, le glutaraldéhyde peut réagir avec les groupes aminés de la gélatine en formant des liaisons imines. Ainsi, le glutaraldéhyde est transformé en s'insérant dans le réseau de gélatine.

Si l'émulsion huile dans l'eau a été atomisée à l'étape b) du procédé selon l'invention, l'ajout d'au moins un agent de réticulation de l'étape c) peut être réalisé dans un lit fluidisé à une température comprise entre 55°C et 65°C, de préférence à 60°C.

Si l'émulsion huile dans l'eau obtenue à l'issue de l'étape a) a été dispersée au cours de l'étape b) du procédé selon l'invention dans une huile alimentaire de manière à obtenir une double émulsion, alors l'étape c) du procédé selon l'invention peut consister à abaisser la température du mélange (ou autrement dit de la double émulsion) obtenu à l'issue de l'étape b) à une température de refroidissement qui est inférieure à la température de transition de phase de la protéine, de manière à solidifier les gouttelettes de principes actifs dans l'huile alimentaire et donc à obtenir une dispersion de granulés de principes actifs humides (à savoir qu'ils comprennent entre 40 et 60% d'eau) dans l'huile alimentaire à laquelle on ajoute à cette température de refroidissement l'agent de réticulation de la protéine.

Par exemple, lorsque la protéine utilisée à l'étape a) est de la gélatine, la température est abaissée à une température comprise entre 12°C et 18°C.

Si l'étape b) du procédé selon l'invention a consisté en une atomisation, la majeure partie de l'eau de l'émulsion a été évaporée au cours de cette étape b), c'est pourquoi l'étape d) du procédé selon l'invention peut consister en un séchage sur lit fluidisé à une température comprise entre 55°C et 65°C, de préférence à 60°C de manière à achever l'évaporation de l'eau, et ce de manière à récupérer une poudre de principes actifs.

Si l'étape b) du procédé selon l'invention a consisté en la préparation d'une double émulsion, alors l'étape d) de récupération des principes actifs peut consister en les étapes successives suivantes :
- on essore les granulés de principes actifs ainsi obtenus à l'issue de l'étape c),
- éventuellement, on absorbe lesdits granulés de principes actifs sur un agent anti-agglomérant tel que de la silice, du stéarate de magnésium ou de l'amidon, de la maltodextrine ou de la fécule de maïs,
- on sèche lesdits granulés de principes actifs dans un lit fluidisé à une température comprise entre 55°C et 65°C, de préférence à 60°C,
et ce de manière à récupérer des particules de principes actifs.

On utilise préférentiellement comme agent anti-agglomérant de la silice.

Avantageusement, la taille des particules récupérées à l'issue de l'étape d) est comprise entre 50 et 630 µm.

Les particules de principes actifs ainsi obtenues à l'issue du procédé de préparation selon l'invention ont une teneur en eau en poids comprise entre 1 et 5%.

Les particules de principes actifs obtenues selon le procédé selon l'invention présentent une insolubilité dans l'eau chaude à 60°C.

Il est à noter que l'ensemble des étapes du procédé de préparation de particules de principes actifs selon l'invention peut être réalisé selon un procédé continu, par exemple à l'aide de réacteurs en cascade dont le remplissage se fait par débordement du réacteur en amont vers le réacteur en aval ou par un procédé discontinu. Il est bien évident qu'un procédé continu présente des avantages économiques notables.

L'invention concerne aussi des particules de principes actifs liposolubles, en particulier pharmaceutiques et/ou alimentaires, susceptibles d'être obtenues par le procédé selon la présente invention.

Un autre objet de l'invention est un pré-mélange, en particulier un pré-mélange agressif, qui comprend des particules de principes actifs liposolubles selon la présente invention.

Le pré-mélange agressif peut comprendre outre des vitamines des constituants tels que choisis parmi le sulfate de cuivre, le carbonate de calcium, le sulfate de cobalt, le sulfate de fer, le carbonate de fer, le iodate de calcium, l'oxyde vert de manganèse, l'oxyde de zinc, des dérivés du sélénium.

L'invention concerne aussi des aliments ou des fourrages qui contiennent des particules de principes actifs liposolubles selon l'invention.

L'invention concerne aussi une composition alimentaire comprenant des particules de principes actifs liposolubles susceptibles d'être obtenues par le procédé de préparation selon la présente invention. Il peut s'agir de compléments alimentaires, qui comprennent par exemple des vitamines, et qui sont destinés à compléter les apports quotidiens nécessaires au bon fonctionnement de l'organisme.
La figure 1 représente un histogramme exprimant le taux de récupération de l'acétate de vitamine A respectivement à 14 et 28 jours des essais n°1 à 11.
La figure 2 représente un histogramme exprimant le taux de récupération du propionate de vitamine A respectivement à 14 et 28 jours des essais n°12 à 14.

### Partie expérimentale :

### Evaluation de la stabilité de particules préparées selon le procédé selon l'invention dans un pré-mélange, dit « pré-mélange agressif » :

Dans le cadre de cette partie expérimentale, le principe actif « testé » a été :
- l'acétate de vitamine A (partie I), puis
- le propionate de vitamine A (partie II),
à savoir des composés sensibles aux conditions de procédé industriels décrites ci-dessus, ainsi qu'en mélange dans un pré-mélange agressif.

Il est à noter que dans les mélanges décrits ci-dessous, des vitamines font partie des constituants des compositions. Il n'est en effet pas nécessaire de préparer selon le procédé selon l'invention toutes les vitamines entrant dans la composition d'un pré-mélange, en particulier celles qui ne sont pas trop sensibles à des conditions extrêmes, et ce afin de limiter le coût de production du pré-mélange.

### I - Principe actif testé : Acétate de vitamine A :

### A- Préparation du principe actif acétate de vitamine A:

Pour tous les essais 1 à 11, dans un mélangeur, on a dissous dans de l'eau, à 50°C, sous agitation à une vitesse de 2 à 3 m/s, de la gélatine, et ce pendant environ 20 minutes.

On a alors ajouté dans ce premier mélangeur, toujours sous agitation à 2 à 3 m/s, un sucre, de l'acétate de vitamine A, du ditertiobutyl-,5,hydroxy-4-toluène (BHT).

On a en outre ajouté pour seulement les essais n°4 à 11 du NaH₂PO₄.

La température du mélangeur était toujours maintenue à 50°C, et ce jusqu'à obtention d'un mélange homogène.

Dans ce mélange homogène ainsi obtenu, l'eau représentait en poids environ 50% dudit mélange.

L'émulsion obtenue aux essais 1 à 11 a ensuite été dispersée dans de l'ester méthylique d'huile de colza.

Puis, pour les essais 1 à 10, la température du mélangeur a été abaissée à une température comprise entre 12°C et 18°C, et l'on a ajouté du glutaraldéhyde comme agent de réticulation de la protéine. On a ainsi obtenu des granulés humides d'acétate de vitamine A.

On a ensuite essoré ces granulés humides et on les a absorbés sur de la silice, puis séchés dans un lit fluidisé à une température de 60°C. On a alors récupéré dix poudres d'acétate de vitamine A d'essais 1 à 10 (ou autrement dit des particules d'acétate de vitamine A d'essais 1 à 10).

En ce qui concerne l'essai n°11, après que l'émulsion obtenue à partir du mélange des constituants de cet essai a été dispersée dans de l'ester méthylique d'huile de colza, on a essoré ces granulés humides et on les a absorbés sur de la silice, puis séchés dans un lit fluidisé à une température de 60°C. Ensuite, on a effectué une réticulation thermique à 100°C. On a ainsi obtenu une poudre d'acétate de vitamine A de cet essai n°11.

Dans le tableau 1 ci-dessous sont récapitulés la nature et la quantité des différents constituants dont sont constituées les poudres des essais n°1 à 11 obtenues de la manière telle que décrite ci-dessus. Les pourcentages sont exprimés en poids par rapport à la composition totale.

Il est à noter qu'un sous-total correspondant à la somme des pourcentages de :
- principe actif,
- antioxydant,
- protéine,
- sucre,
- éventuellement sel inorganique,
est exprimé.

Les autres constituants de ces poudres d'essais 1 à 11 sont la silice utilisée lors de l'absorption, l'eau résiduelle après séchage, l'huile utilisée, à savoir l'ester méthylique d'huile de colza, pour réaliser la double émulsion. Les pourcentages exprimés en poids de ces trois derniers constituants sont respectivement de l'ordre de 2, 3 et 2%, selon les différents essais. C'est pourquoi, il est fait mention dans le tableau 1 de « qsp % [silice + eau + ester méthylique d'huile de colza] ».

De plus, comme expliqué ci-dessus en ce qui concerne l'agent de réticulation de la protéine, le glutaraldéhyde réagit avec les groupes aminés de la gélatine en formant des liaisons imines. Le glutaraldéhyde est donc transformé, et c'est pourquoi, on ne retrouve pas ce constituant dans le tableau 1, qui récapitule la composition finale des particules obtenues à l'issue du procédé de préparation.

Les essais 1 à 3 sont des essais comparatifs. En effet, comme décrit ci-dessus, il n'a pas été ajouté de NaH₂PO₄ dans ces essais 1 à 3.

Les essais 4 à 10 ont été réalisés selon la présente invention.

Par ailleurs, à la différence des essais 1 à 10, l'antioxydant utilisé pour l'essai n°11 était l'éthoxyquine.

L'essai n°11 est aussi un essai comparatif par rapport à la présente invention, étant donné que pour cet essai une réticulation thermique de la protéine a été mise en oeuvre, à la différence des essais 1 à 10 pour lesquels on a procédé à une réticulation chimique de la protéine (à savoir par l'ajout de glutaraldéhyde).

**Tableau 1 : Pourcentages des différents constituants en fonction de l'essai.**

| **N°Essai % Constituants** | | **N° 1** | **N°2** | **N°3** | **N°4** | **N°5** | **N°6** | **N°7** | **N°8** | **N°9** | **N°10** | **N°11** |
|---|---|---|---|---|---|---|---|---|---|---|---|---|
| **Principe actif** | acétate de vitamine A | 38,5 | 39,0 | 37,7 | 38,3 | 38,3 | 36,5 | 38,3 | 39,2 | 38,3 | 37,2 | 37,2 |
| **antioxydant** | BHT | 10,0 | 20,5 | 28,3 | 17,8 | 17,8 | 17 | 18,5 | 18,3 | 22,6 | 23,1 | ***4*** |
| **Protéine** | Gélatine 140 BI | 21,7 | 0 | 0 | 0 | 0 | 0 | 0 | 21,9 | 0 | 23,3 | 35,1 |
| | Gélatine 220 BI | 0 | 0 | 0 | 0 | 18,5 | 17,6 | 18,5 | 0 | 0 | 0 | 0 |
| | Gélatine 300 BI | 0 | 19,5 | 17,9 | 18,5 | 0 | 0 | 0 | 0 | 17,9 | 0 | 0 |
| **Sucre** | Sirop de glucose | 23,5 | 14,7 | 9,9 | 14,0 | 14 | 13,3 | 14 | 9,3 | 10 | 0 | 0 |
| | Méliose | 0 | 0 | 0 | 0 | 0 | 0 | 0 | 0 | 0 | 5 | 8,1 |
| **Sel inorganique** | NaH₂PO₄ | 0 | 0 | 0 | **5,2** | **5,2** | **4,9** | **5,2** | **4,8** | **5** | **5** | **5** |
| **Sous-total** | | **93,7** | **93,7** | **93,8** | **93,8** | **93,8** | **94,3** | **94,5** | **93,5** | **93,8** | **93,6** | **94,4** |
| **Total** | | **qsp % [silice + eau + ester méthylique d'huile de colza]** | | | | | | | | | | |

Toutes les poudres des essais 1 à 11 ont ensuite été mélangées dans un pré-mélange dit « agressif » à 6000 u.i./g d'acétate de vitamine A qui a été préparé tel que détaillé ci-après.

### B- Préparation d'un pré-mélange oligoporc :

Un pré-mélange dit « oligoporc », à savoir destiné à la nutrition porcine, a été préparé en mélangeant ensemble les constituants suivants (les pourcentages indiqués représentent les pourcentages en poids par rapport à la composition totale du pré-mélange oligoporc) :
- sulfate de cobalt : 0,39% ;
- sulfate de cuivre : 10% ;
- sulfate de fer : 12,5% ;
- carbonate de fer : 20% ;
- iodate de calcium : 0,15% ;
- oxyde vert de manganèse : 10,7% ;
- oxyde de zinc : 24,9% ;
- un dérivé de sélénium (nom commercial : SELENIPHOS): 0,60% ;
- qsp % de carbonate de calcium.

### C- Préparation d'un complexe :

Un mélange de principes actifs, ci-après dénommé « complexe » a été préparé de la manière suivante (les quantités sont exprimées en g) :
Les constituants suivants ont été mélangés ensemble :
- vitamine D3 à 500 u.i./g : 0,400 g
- vitamine E à 50% : 3,840 g
- vitamine B1 : 0,128 g
- vitamine B2 : 0,384 g
- panthothénate de calcium : 0,960 g
- vitamine K3 à 96% : 0,128 g
- vitamine B6 : 0,128 g
- vitamine B12 à 0,1% : 1,280 g
- niacine : 1,920 g
- vitamine B9 : 0,064 g
- carbonate de calcium : 3,750 g
- remoulage : 1,600 g

### D- Préparation d'un pré-mélange agressif à 6 000 u.i. / g :

Un pré-mélange agressif a été préparé en mélangeant ensemble les constituants suivants (les quantités sont exprimées en g) :
- pré-mélange oligoporc (décrit ci-dessus) : 24,0 g
- chlorure de choline à 50 % : 38,4 g
- sulfate de cuivre : 22,4 g
- carbonate de calcium : 59,2 g
- complexe (décrit ci-dessus) : 14,6 g

Soit un mélange d'un poids total de 158,6 g, auquel a été ajouté 0,95 g de poudre d'acétate de vitamine A telle que préparée ci-dessus en fonction des essais 1 à 11.

Afin de démontrer les bonnes propriétés de stabilité d'une poudre d'acétate de vitamine A préparée selon le procédé selon l'invention (essais 4 à 10), le taux de récupération de cet acétate de vitamine A a été mesuré au bout 14 et 28 jours, et comparé à celui mesuré pour une poudre d'acétate de vitamine A non préparée selon l'invention (essais 1 à 3 et 11).

Pour effectuer ces mesures du taux de récupération, les poudres des essais 1 à 11 ont été placées dans une enceinte, maintenue à une température de 30°C et sous une humidité relative de 75%.

Les analyses de la teneur en acétate de vitamine A ont été réalisées après extraction, puis par dosage par Chromatographie Liquide à haute performance (CLHP) :
- chromatographe isocratique équipé d'une pompe pouvant délivrer 1,2 mL/min,
- vanne d'injection de type RHEODYNE,
- détecteur UV,
- Colonne : Lichrocart 125-4, Lichrosorb Si 60 (5µm),
- phase mobile : n-hexane (99,5%) / 2-propanol (0,05%).

Ainsi, plus le pourcentage du taux de récupération est élevé, plus cela est significatif que l'acétate de vitamine A n'a pas été dégradé dans le pré-mélange agressif. Le tableau 2 récapitule les résultats obtenus.

**Tableau 2 exprimant les taux de récupération de l'acétate de vitamine A à 14 et 28 jours en fonction des essais 1 à 11.**

| **Taux de récupération (%)** | **N°1** | **N°2** | **N°3** | **N°4** | **N°5** | **N°6** | **N°7** | **N°8** | **N°9** | **N°10** | **N°11** |
|---|---|---|---|---|---|---|---|---|---|---|---|
| **14 jours** | 56 | 62 | 50 | 90 | 69 | 74 | 62 | 70 | 81 | 71 | 70 |
| **28 jours** | 24 | 42 | N.D. | 67 | 52 | 60 | 48 | 47 | 56 | 57 | 36 |

Le taux de récupération de l'essai n°3 n'a pas été mesuré à 28 jours.

Ce tableau 2 témoigne de la bonne stabilité des poudres d'acétate de vitamine A obtenues selon le procédé de préparation de l'invention par rapport aux essais comparatifs 1 à 3 et 11.

A 28 jours, le taux de récupération des essais selon l'invention est toujours supérieur à celui des essais comparatifs.

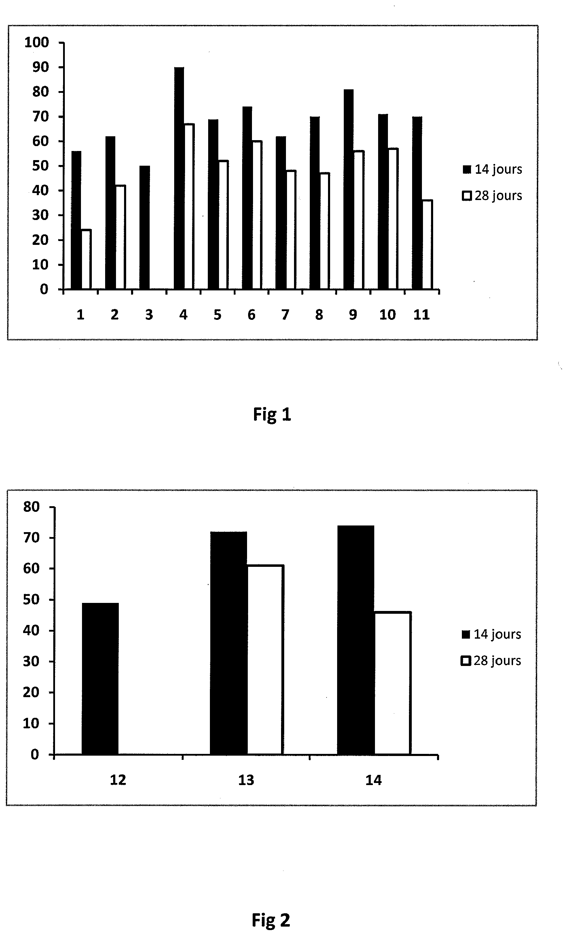

La figure 1 représente un histogramme exprimant le taux de récupération de l'acétate de vitamine A respectivement à 14 et 28 jours des essais n°1 à 11 qui sont indiqués dans le tableau 2 ci-dessus.

### II - Principe actif testé : propionate de vitamine A :

### E- Préparation du principe actif propionate de vitamine A:

Le principe actif propionate de vitamine A a été préparé de la même façon que l'acétate de vitamine A tel que décrit ci-dessus. La nature et la quantité des différents constituants des essais n°12 à 14 sont récapitulées dans le tableau 3 ci-dessous et sont exprimées en pourcentages en poids par rapport à la composition totale.

**Tableau 3 : Pourcentages des différents constituants en fonction de l'essai. L'essai n°12 est un essai comparatif. En effet, comme décrit ci-dessus, il n'a pas été ajouté de NaH₂PO₄ dans cet essai.**

| **Constituant** | **Essai** | **N° 12** | **N°13** | **N°14** |
|---|---|---|---|---|
| **Principe actif** | Propionate de vitamine A | 40,2 | 39,9 | 39,9 |
| **Antioxydant** | BHT | 9,8 | 9,4 | 10,3 |
| **Protéine** | Gélatine 140 BI | 0 | 0 | 21,5 |
| | Gélatine 220 BI | 21,7 | 21,6 | 0 |
| | Gélatine 300 BI | 0 | 0 | 0 |
| **Sucre** | Sirop de glucose | 23,6 | 19,1 | 0 |
| | Méliose | 0 | 0 | 19,1 |
| **Sel inorganique** | NaH₂PO₄ | 0 | 4,9 | 3 |
| **Sous-total** | | **94,9** | **94,9** | **93,8** |
| **Total** | | **qsp % [silice + eau + ester méthylique d'huile de colza]** | | |

Les essais n°13 et 14 ont été réalisés selon la présente invention.

Le même pré-mélange agressif que pour l'acétate de vitamine A a été préparé. On a donc obtenu un mélange tel que décrit ci-dessus d'un poids de 158,6 g auquel a été ajouté 0,95 g de poudre de propionate de vitamine A tel que préparé ci-dessus en fonction des essais 12 à 14.

Pour les essais 12 à 14, le taux de récupération du proponiate de vitamine A a été mesuré selon le même protocole que celui décrit pour les essais 1 à 11.

Le tableau 4 ci-dessous récapitule les résultats obtenus en ce qui concerne le taux de récupération du proponiate de vitamine A.

**Tableau 4 exprimant les taux de récupération de propionate de vitamine A à 14 et 28 jours en fonction des essais 12 à 14.**

| **Taux de récupération (%)** | **N°12** | **N°13** | **N°14** |
|---|---|---|---|
| **14 jours** | 49 | 72 | 74 |
| **28 jours** | N.D. | 61 | 46 |

Le taux de récupération de l'essai n°12 n'a pas été mesuré à 28 jours.

On constate un meilleur taux de récupération du propionate de vitamine A à 14 jours pour les essais selon l'invention que pour l'essai comparatif.

La figure 2 représente un histogramme exprimant le taux de récupération du propionate de vitamine A respectivement à 14 et 28 jours des essais n°12 à 14 et qui sont indiqués dans le tableau 4 ci-dessus.

### Evaluation de la solubilité de particules de principes actifs obtenues selon le procédé selon l'invention :

La solubilité des poudres obtenues (ou autrement dit des particules de principes actifs) dans les essais n°1 à 14 a été testée dans de l'eau chaude à 90°C.

On a constaté que les essais préparés selon l'invention sont insolubles dans de l'eau chaude à 90°C.

L'essai comparatif n° 11 qui a été préparé par une réticulation thermique, et donc non chimique est quant à lui soluble dans l'eau chaude à 90°C au bout d'une minute d'immersion dans l'eau chaude.

Ainsi, les particules préparées selon le procédé selon l'invention témoignent non seulement d'une bonne stabilité dans un pré-mélange dit agressif à 14 et 28 jours, mais aussi d'une insolubilité dans une eau chaude à 90°C, et ce à la différence des produits non réticulés chimiquement.

## Revendications

1. Procédé de préparation de principes actifs liposolubles, en particulier pharmaceutiques et/ou alimentaires, sous la forme de particules, **caractérisé en ce que** ledit procédé comprend les étapes suivantes :
a) on prépare une émulsion huile dans l'eau comprenant en pourcentage en poids par rapport au poids total de ladite émulsion :
- 8 à 20 % d'au moins une protéine, préférentiellement 10 à 15%,
- 5 à 15 % d'au moins un sucre, préférentiellement 8 à 12%,
- 10 à 22% d'au moins un principe actif liposoluble sous forme huileuse et/ou dissous dans une huile alimentaire, préférentiellement 15 à 20%,
- qsp % d'eau,
b) on procède au formage de particules sous la forme substantiellement sphériques par la dispersion de l'émulsion huile dans l'eau obtenue à l'issue de l'étape a) dans un fluide,
c) on ajoute au moins un agent de réticulation de la protéine à la dispersion obtenue à l'issue de l'étape b), ledit agent de réticulation étant choisi parmi l'acétaldéhyde, le glutaraldéhyde, le glyoxal, de préférence le glutaraldéhyde,
d) on récupère les principes actifs sous la forme substantiellement sphériques, l'étape de réticulation de la protéine est réalisée sans étape de traitement thermique, **caractérisé en ce que**
ladite émulsion de l'étape a) comprend en outre 0,5 à 3%, préférentiellement 2 à 3%, d'un sel inorganique qui est le NaH₂PO₄.

2. Procédé de préparation selon la revendication 1, **caractérisé en ce que** la au moins une protéine est choisie parmi la gélatine, la caséine ou la caséinate, les protéines du soja ou du maïs.

3. Procédé de préparation selon l'une quelconque des revendications 1 à 2, **caractérisé en ce que** le au moins un sucre est choisi parmi les polyols, les monosaccharides, les disaccharides, les sirops de glucose et de fructose et les maltodextrines.

4. Procédé de préparation selon l'une quelconque des revendications 1 à 3, **caractérisé en ce que** le au moins un principe actif est choisi parmi les vitamines telles que la vitamine A, E, D, K, les dérivés de ces vitamines tels leurs esters, les caroténoïdes tels que le bêta-carotène, le lycopène, la bixine, la zéaxanthine, la citranaxanthine, l'asthaxanthine, la canthaxanthine, la lutéine, la capsanthine, la cryptoxanthine, l'acide bêta-apo-8' caroténoïque et ses esters, le bêta-apo-8' carotenal, le bêta-apo-12'-caroténal, les acides gras polyinsaturés tels que les omégas 3 et omégas 6.

5. Procédé de préparation selon la revendication 4, **caractérisé en ce que** le au moins un principe actif est l'acétate de vitamine A, le propionate de vitamine A, le palmitate de vitamine A, l'acétate de tocophéryl.

6. Procédé de préparation selon l'une quelconque des revendications 1 à 5, **caractérisé en ce que** préalablement à l'étape a) dudit procédé, on dissout le au moins un principe actif dans une huile alimentaire telle que l'huile d'arachide, de tournesol, de colza, de maïs, de soja, de palme ou de foie de morue.

7. Procédé de préparation selon l'une quelconque des revendications 1 à 6, **caractérisé en ce qu'**à l'étape b) dudit procédé, l'émulsion huile dans l'eau est dispersée dans une huile alimentaire et **en ce que** l'étape c) consiste à abaisser la température du mélange obtenu à l'issue de l'étape b) à une température de refroidissement inférieure à la température de transition de phase de la protéine de manière à obtenir une dispersion de granulés de principes actifs humides dans l'huile alimentaire à laquelle on ajoute à cette température de refroidissement l'agent de réticulation de la protéine.

8. Procédé de préparation selon l'une quelconque des revendications 1 à 6, caractérisé en qu'à l'étape b) dudit procédé, l'émulsion huile dans l'eau obtenue à l'issue de l'étape a) est dispersée dans de l'air par atomisation.

9. Procédé de préparation selon la revendication 7, **caractérisé en ce que** l'étape d) dudit procédé consiste en les étapes successives suivantes :
- on essore les granulés de principes actifs ainsi obtenus à l'issue de l'étape c),
- éventuellement, on absorbe lesdits granulés de principes actifs sur un agent anti-agglomérant,
- on sèche lesdits granulés de principes actifs dans un lit fluidisé, de manière à récupérer des particules de principes actifs.

10. Procédé de préparation selon l'une quelconque des revendications 1 à 9, **caractérisé en ce que** la taille des particules récupérées à l'issue de l'étape d) est comprise entre 50 et 630 µm.

11. Particules de principes actifs liposolubles, en particulier pharmaceutiques et/ou alimentaires, susceptibles d'être obtenues par le procédé selon l'une quelconque des revendications 1 à 10.

12. Pré-mélange, en particulier pré-mélange agressif, comprenant des particules de principes actifs liposolubles selon la revendication 11.

13. Aliment ou fourrage contenant des particules de principes actifs liposolubles selon la revendication 11.

14. Composition alimentaire comprenant des particules de principes actifs liposolubles selon la revendication 11.

## Patentansprüche

1. Verfahren zur Herstellung von fettlöslichen, insbesondere pharmazeutischen und/oder Nahrungsmittelwirkstoffen in Form von Partikeln, **dadurch gekennzeichnet, dass** das Verfahren die folgenden Schritte umfasst:
a) Herstellen einer Öl-in-Wasser-Emulsion, umfassend in Gewichtsprozent mit Bezug auf das Gesamtgewicht der Emulsion:
- 8 bis 20 % mindestens eines Proteins, vorzugsweise 10 bis 15 %,
- 5 bis 15 % mindestens eines Zuckers, vorzugsweise 8 bis 12 %,
- 10 bis 22 % mindestens eines fettlöslichen Wirkstoffs in öliger Form und/oder gelöst in einem Nahrungsmittelöl, vorzugsweise 15 bis 20 %,
- *quantum satis* % Wasser,
b) Bilden von Partikeln in einer im Wesentlichen sphärischen Form durch die Dispersion der öligen Emulsion im Wasser, erhalten am Ausgang von Schritt a) in einem Fluid,
c) Zugeben von mindestens einem Vernetzungsmittel des Proteins zur Dispersion, erhalten am Ausgang von Schritt b), wobei das Vernetzungsmittel ausgewählt ist aus Acetaldehyd, Glutaraldehyd, Glyoxal, vorzugsweise Glutaraldehyd,
d) Wiedergewinnen der Wirkstoffe in der im Wesentlichen sphärischen Form,
wobei der Schritt des Vernetzens des Proteins ohne Schritt des thermischen Behandelns durchgeführt wird, **dadurch gekennzeichnet, dass**
die Emulsion von Schritt a) außerdem 0,5 bis 3 %, vorzugsweise 2 bis 3 %, eines anorganischen Salzes umfasst, wobei es sich um NaH₂PO₄ handelt.

2. Verfahren zur Herstellung nach Anspruch 1, **dadurch gekennzeichnet, dass** das mindestens eine Protein ausgewählt ist aus Gelatine, Casein oder Caseinat, Proteinen von Soja oder von Mais.

3. Verfahren zur Herstellung nach einem der Ansprüche 1 bis 2, **dadurch gekennzeichnet, dass** der mindestens eine Zucker ausgewählt ist aus den Polyolen, Monosacchariden, Disacchariden, Glukose- und Fruktosesirups und Maltodextrinen.

4. Verfahren zur Herstellung nach einem der Ansprüche 1 bis 3, **dadurch gekennzeichnet, dass** der mindestens eine Wirkstoff ausgewählt ist aus den Vitaminen wie z. B. Vitamin A, E, D, K, den Derivaten dieser Vitamine wie z. B. ihren Estern, Carotenoiden wie z. B. Betacaroten, Lycopin, Bixin, Zeaxanthin, Zitranaxanthin, Asthaxanthin, Canthaxanthin, Lutein, Capsanthin, Cryptoxanthin, Beta-apo-8'-Carotenoidsäure und ihren Estern, Beta-apo-8'-Carotinal, Beta-apo-12'-Carotinal, mehrfach ungesättigten Fettsäuren wie z. B. Omega 3 und Omega 6.

5. Verfahren zur Herstellung nach Anspruch 4, **dadurch gekennzeichnet, dass** der mindestens eine Wirkstoff Vitamin A-Acetat, Vitamin A-Propionat, Vitamin A-Palmitat, Tocopherolacetat ist.

6. Verfahren zur Herstellung nach einem der Ansprüche 1 bis 5, **dadurch gekennzeichnet, dass** vor Schritt a) des Verfahrens der mindestens eine Wirkstoff in einem Nahrungsmittelöl wie z. B. Erdnussöl, Sonnenblumenöl, Rapsöl, Maisöl, Sojaöl, Palmöl oder Lebertran gelöst wird.

7. Verfahren zur Herstellung nach einem der Ansprüche 1 bis 6, **dadurch gekennzeichnet, dass** in Schritt b) des Verfahrens die Öl-in-Wasser-Emulsion in einem Nahrungsmittelöl dispergiert wird, und dadurch, dass Schritt c) darin besteht, die Temperatur der Mischung, erhalten am Ausgang von Schritt b), auf eine Kühltemperatur unter der Übergangsphasentemperatur des Proteins zu senken, um eine Dispersion von feuchtem Granulat von Wirkstoffen im Nahrungsmittelöl zu erhalten, der bei dieser Kühltemperatur das Vernetzungsmittel des Proteins zugegeben wird.

8. Verfahren zur Herstellung nach einem der Ansprüche 1 bis 6, **dadurch gekennzeichnet, dass** in Schritt b) des Verfahrens die Öl-in-Wasser-Emulsion, erhalten am Ausgang von Schritt a) durch Zerstäubung in Luft dispergiert wird.

9. Verfahren zur Herstellung nach Anspruch 7, **dadurch gekennzeichnet, dass** Schritt d) des Verfahrens aus den folgenden sukzessiven Schritten besteht:
- Schleudern des so erhaltenen Granulats von Wirkstoffen am Ausgang von Schritt c),
- eventuell Absorbieren des Granulats von Wirkstoffen auf einem Trennmittel,
- Trocknen des Granulats von Wirkstoffen in einer Wirbelschicht, um die Partikel von Wirkstoffen wiederzugewinnen.

10. Verfahren zur Herstellung nach einem der Ansprüche 1 bis 9, **dadurch gekennzeichnet, dass** die Größe der am Ausgang von Schritt d) wiedergewonnenen Partikel im Bereich zwischen 50 und 630 µm liegt.

11. Partikel von fettlöslichen, insbesondere pharmazeutischen und/oder Nahrungsmittelwirkstoffen, die durch das Verfahren nach einem der Ansprüche 1 bis 10 erhalten werden können.

12. Vormischung, insbesondere aggressive Vormischung, umfassend Partikel von fettlöslichen Wirkstoffen nach Anspruch 11.

13. Nahrungsmittel oder Futter, enthaltend Partikel von fettlöslichen Wirkstoffen nach Anspruch 11.

14. Nahrungsmittelzusammensetzung, umfassend Partikel von fettlöslichen Wirkstoffen nach Anspruch 11.

## Claims

1. A method for preparing liposoluble active substances, in particular pharmaceutical and/or feed active substances, in the form of particles, **characterized in that** said method comprises the following steps of:
a) preparing an oil-in-water emulsion comprising in weight percentage with respect to the total weight of said emulsion:
- 8 to 20 % of at least one protein, preferably 10 to 15 %,
- 5 to 15 % of at least one sugar, preferably 8 to 12 %,
- 10 to 22 % of at least one liposoluble active substances in an oily form and/or dissolved in an edible oil, preferably 15 to 20 %,
- Q.S. of water,
b) proceeding with forming of particles in a substantially spherical shape by the dispersion of the oil-in-water emulsion obtained on completion of step a) in a fluid,
c) adding at least one cross-linking agent of the protein to the dispersion obtained on completion of step b), said cross-linking agent being selected from acetaldehyde, glutaraldehyde, glyoxal, preferably glutaraldehyde,
d) recovering the active substances in a substantially spherical shape,
the step of cross-linking the protein is carried out without any heat treatment step, **characterized in that**
said emulsion of step a) further comprises 0.5 to 3 %, preferably 2 to 3 %, of an inorganic salt which is NaH₂PO₄.

2. The preparation method according to claim 1, **characterized in that** the at least one protein is selected from gelatin, casein, caseinate, soy or corn proteins.

3. The preparation method according to any one of claims 1 to 2, **characterized in that** the at least one sugar is selected from polyols, monosaccharides, disaccharides, glucose and fructose syrups and maltodextrins.

4. The preparation method according to any one of claims 1 to 3, **characterized in that** the at least one active substance is selected from vitamins such as vitamins A, E, D, K, the derivatives of the these vitamins such as the esters thereof, carotenoids such as beta-carotene, lycopene, bixin, zeaxanthin, citranaxanthin, astaxanthin, canthaxanthin, lutein, capsanthin, cryptoxanthin, beta-apo-8'-carotenoic acid and the esters thereof, beta-apo-8'-carotenal, beta-apo-12'-carotenal, polyunsaturated fatty acids such as omegas 3 and omegas 6.

5. The preparation method according to claim 4, **characterized in that** the at least one active substance is vitamin A acetate, vitamin A propionate, vitamin A palmitate, tocopheryl acetate.

6. The preparation method according to any one of claims 1 to 5, **characterized in that** prior to step a) of said method, the at least one active substance is dissolved in an edible oil such as peanut, sunflower, colza, corn, soy, palm or cod liver oil.

7. The preparation method according to any one of claims 1 to 6, **characterized in that** at step b) of said method, the oil-in-water emulsion is dispersed in an edible oil and **in that** step c) consists in lowering the temperature of the mixture obtained on completion of step b) to a cooling temperature lower than the phase transition temperature of the protein so as to obtain a dispersion of humid granules of active substances in the edible oil to which the cross-linking agent of the protein is added at this cooling temperature.

8. The preparation method according to any one of claims 1 to 6, **characterized in that** at step b) of said method, the oil-in-water emulsion obtained on completion of step a) is dispersed in the air by atomization.

9. The preparation method according to claim 7, **characterized in that** step d) of said method consists in the following successive steps of:
- drying out the granules of active substances obtained on completion of step c),
- possibly, absorbing said granules of active substances on an anticaking agent,
- drying said granules of active substances in a fluidized bed, so as to recover particles of active substances.

10. The preparation method according to any one of claims 1 to 9, **characterized in that** the size of the particles recovered on completion of step d) is comprised between 50 and 630 µm.

11. Particles of liposoluble active substances, in particular pharmaceutical and/or feed active substances, likely to be obtained by the preparation method according to any one of claims 1 to 10.

12. A pre-mixture, in particular an aggressive pre-mixture, comprising particles of liposoluble active substances according to claim 11.

13. A foodstuff or fodder containing particles of liposoluble active substances according to claim 11.

14. A feed composition comprising particles of liposoluble active substances according to claim 11.
